# FASCICULE DE BREVET EUROPEEN

(11) **EP 3 344 204 B1**
(45) Date de publication et mention de la délivrance du brevet: **25.12.2019**
(21) Numéro de dépôt: 15775251.0
(22) Date de dépôt: 01.09.2015
(51) Int. Cl.: A61F 9/02

(54) **ACCESSOIRE DE PROTECTION VISUELLE MUNI DE MOYENS D'ÉCARTEMENT DE L'ÉCRAN VIS-À-VIS DE LA MONTURE**
SICHTSCHTUTZZUBEHÖR MIT VORRICHTUNG ZUR BEABSTANDUNG DES BILDSCHIRMS VOM RAHMEN
VISUAL PROTECTION ACCESSORY PROVIDED WITH MEANS FOR SPACING THE SCREEN AWAY FROM THE FRAME

(43) Date de publication de la demande: 11.07.2018
(73) Titulaire: Société Julbo, 39400 Longchaumois (FR)
(72) Inventeur: BONNET-MATHIEU, Clement, 39220 Les Rousses (FR)
(74) Mandataire: Delaveau, Sophie
(86) Numéro de dépôt international: PCT/FR2015/000179
(87) Numéro de publication internationale: WO 2017/037350

(56) Documents cités:
- WO-A1-01/89333
- FR-A1- 2 655 432
- US-A1- 2004 083 540
- US-A1- 2011 225 709

## Description

L'invention concerne principalement un accessoire de protection visuelle. L'invention trouve notamment application pour la réalisation d'un masque de ski.

L'invention s'inscrit dans le domaine des accessoires et équipements de protection visuelle. Les équipements de protection visuelle comportent généralement au moins une monture sur laquelle est monté un écran et trouvent application dans différents domaines tels que certains domaines professionnels dans lesquels ces équipements assurent une protection vis-à-vis de projections, ou encore dans le domaine sportif comme c'est le cas pour les masques de ski.

Un problème commun aux accessoires de protection visuelle réside dans l'absence ou une mauvaise aération qui peut conduire à un dépôt de buée sur la face interne de l'écran.

C'est notamment le cas pour les masques de ski. Ces derniers prévoient généralement des lumières ménagées sur la monture qui sont recouvertes de mousse autorisant ainsi l'air extérieur à pénétrer dans le masque tout en évitant que la neige n'entre dans le masque. Si l'aération est suffisante en descente à grande vitesse face au vent, elle s'avère insuffisante dans les phases d'attente ou de moindre vitesse.

Les utilisateurs choisissent pour ces raisons d'utiliser des montures plus légères pour les phases d'attente ou les montées, et s'équipent d'un masque avant de commencer les descentes. Mais ce changement d'équipement est peu pratique pour l'utilisateur.

Il existe des masques qui prévoient un système de ventilation électrique mais ce système est encombrant, lourd, et présente une autonomie limitée.

Il existe également des masques qui prévoient plusieurs ouvertures pour augmenter le flux d'air entrant, mais l'efficacité de ventilation de ces masques reste insuffisance.

Un accessoire de protection visuelle tel que celui du préambule de la revendication 1 es connu du document US-A-2004/0083540.

Dans ce contexte, la présente invention vise un accessoire de protection visuelle offrant une aération supérieure aux dispositifs existant et mettant en œuvre des moyens d'activation de l'aération simples pour l'utilisateur.

A cet effet, l'accessoire de protection visuelle de l'invention comporte une monture sur laquelle est monté un écran, et est caractérisé en ce qu'il comporte des moyens d'écartement de l'écran vis-à-vis de la monture aptes à faire adopter à l'écran une première position d'utilisation dans laquelle l'écran est en appui de contact sur la monture, et une seconde position d'aération dans laquelle toute la surface de l'écran est mise à distance de la monture du côté opposé au visage de l'utilisateur.

L'équipement de l'invention peut également comporter les caractéristiques optionnelles suivantes considérées isolément ou selon toutes les combinaisons techniques possibles :
- l'accessoire comporte deux pièces pivot situées respectivement au niveau du bord latéral gauche et du bord latéral droit du dit accessoire, chacune des pièces pivot étant montée d'une part sur l'écran, et étant montée d'autre part pivotante relativement à la monture.
- chacune des deux pièces pivot comporte une première charnière supérieure et une première charnière inférieure de liaison pivotante à la monture.
- chacune des deux pièces pivot est montée également pivotante relativement à l'écran.
- chacune des deux pièces pivot comporte une seconde charnière supérieure et une seconde charnière inférieure de liaison pivotante à l'écran.
- l'accessoire comporte deux pièces de liaison situées respectivement au niveau du bord latéral gauche et du bord latéral droit de l'écran en étant d'une part solidarisé à l'écran et d'autre part en liaison pivotante avec la pièce pivot associée.
- l'accessoire comporte des moyens de maintien fixe de l'écran contre la monture en position d'utilisation, les dits moyens de maintien fixe comprenant au moins un bosselage ménagé sur la monture ou sur la pièce de liaison qui coopère en encliquetage avec une gorge ménagée sur la monture ou la pièce de liaison.
- dans la seconde position d'aération, l'écran est à une distance de la monture comprise entre 5 et 10 millimètres.

Enfin, l'invention trouve notamment application dans la fabrication d'un masque de ski comportant un tel accessoire.

D'autres caractéristiques et avantages de l'invention ressortiront clairement de la description qui en est donnée ci-dessous, à titre indicatif et nullement limitatif, en référence aux figures annexées parmi lesquelles :

- la figure 1 est une représentation schématique en perspective et en élévation de l'accessoire de protection visuelle de l'invention représentée dans sa première position d'utilisation et pour lequel seule la partie en plastique souple de la pièce d'appui sur le visage est illustrée,
- la figure 2 est une représentation schématique en perspective et en élévation de l'accessoire de protection visuelle de l'invention représentée dans sa seconde position d'aération,
- la figure 3 est une vue agrandie de la partie III de la figure 2, et
- la figure 4 est une représentation schématique de coté de l'accessoire de protection visuelle de l'invention représentée dans sa seconde position d'aération.

Dans la description qui suit les références faites à « l'arrière » du masque s'adressent au côté du visage de l'utilisateur et à l'inverse, les références faites à « l'avant » du masque s'adressent au côté opposé au visage de l'utilisateur.

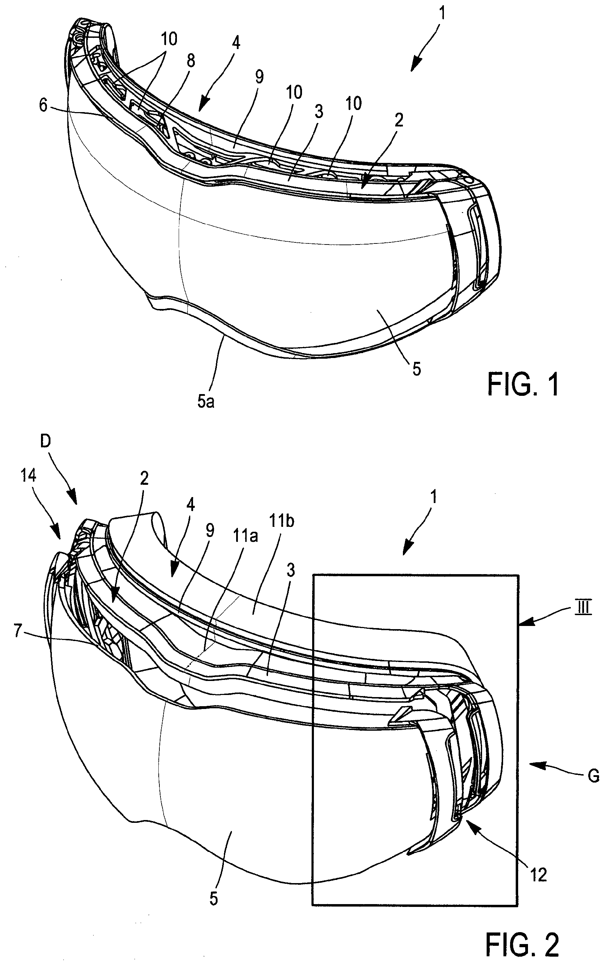

Le masque de ski 1 représenté partiellement sur la figure 1 comporte une monture rigide 2 faite essentiellement d'une bordure périphérique 3 qui s'adapte en partie supérieure au front de l'utilisateur et qui comporte en partie inférieure un décroché 5a d'ajustement sur le nez de l'utilisateur.

Un écran 5 est monté à l'avant de la monture 2. La découpe de l'écran 5 s'ajuste au bord périphérique 3 de la monture 2 tout en prévoyant un jeu 6 entre le bord périphérique 7 de l'écran et le bord périphérique 3 de la monture 2 pour autoriser une entrée d'air dans le masque 1 lorsque l'écran 5 est dans sa première position d'utilisation telle que représentée sur la figure 1 et comme il sera expliqué plus en détail plus loin.

Le masque 1 comporte également une pièce d'appui sur le visage 4 réalisée en un matériau souple, et qui est solidarisé par exemple par collage ou sur-injection à la face arrière 8 de la monture 2.

La pièce d'appui 4 est faite d'un cadre élastique en plastique 9 (figure 1) et comporte sur sa tranche supérieure une série de lumières 10 recouverte d'une fine couche de mousse 11a d'épaisseur d'environ 2 millimètres (figure 2). La pièce d'appui 4 comporte également un couche de mousse 11b d'épaisseur d'environ 12 millimètres qui est collée sur l'arrière du cadre 9 et destiné à venir en appui sur le visage de l'utilisateur.

Le jeu 6 entre le bord périphérique 7 de l'écran et le bord périphérique 3 de la monture 2 et la série de lumières 10 du cadre élastique 9 sont en communication, ce qui permet à un flux d'air extérieur de pénétrer dans le masque 1 lorsque ce dernier est dans sa première position d'utilisation, c'est-à-dire dans la position dans laquelle l'écran 3 est en appui de contact sur la monture 2.

Il est également prévu de façon connue mais non représentée une bride d'accroche solidaire du masque, et en particulier solidaire du cadre élastique 9 de la pièce d'appui 4, qui est destinée à enserrer la tête de l'utilisateur pour assurer le positionnement fixe du masque 1 sur le visage.

Le masque 1 présente une forme générale convexe qui permet de s'adapter à toute la partie supérieure du visage de l'utilisateur.

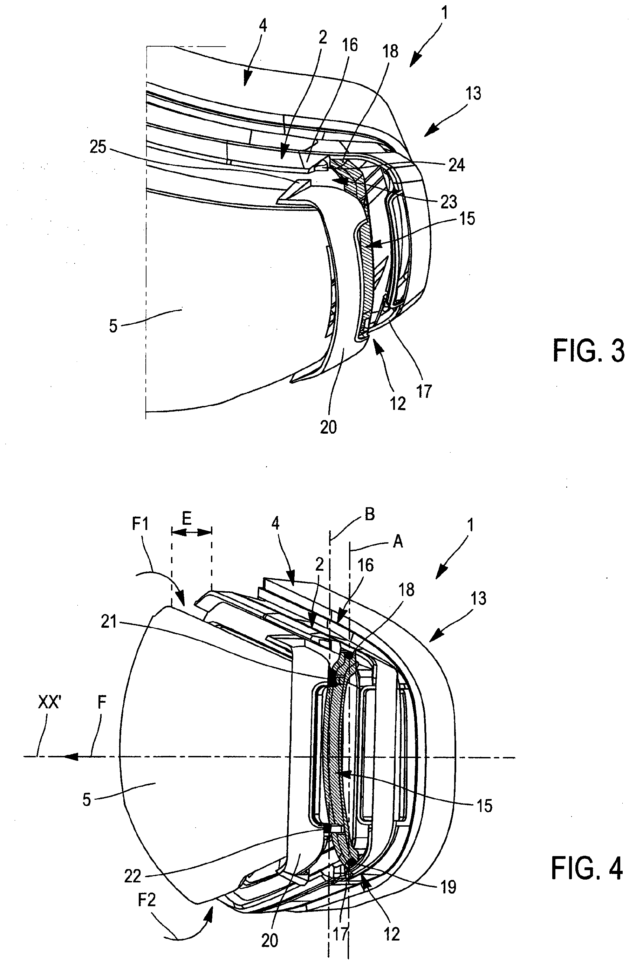

Selon l'invention, le masque de ski 1 comporte des moyens d'écartement 12 permettant de faire passer l'écran 5 de sa première position d'utilisation en appui de contact sur la monture 2 (figure 1), à une position d'aération dans laquelle l'intégralité de la surface de l'écran 2 est mise à distance de la monture 2 par le jeu d'une translation vers l'avant du masque 1 selon l'axe XX' représenté sur la figure 4

Les figures 3 et 4 illustrent les moyens d'écartement 12 situés sur le côté gauche G du masque 1 qui s'appliquent *mutatis mutandis* aux moyens d'écartement identiques non visibles sur les figures et situés sur le côté droit D du masque 1.

Les moyens d'écartement 12 prévoient ainsi au niveau des bords latéraux gauche 13 et droit 14 une pièce pivot 15 solidaire d'une part de la monture 2, et d'autre part de l'écran 5.

La pièce pivot 15 présente une forme de C lorsqu'elle est considérée sur le côté conformément à la vue de la figure 4.

Les extrémités libres de la pièce pivot 15 s'étendent entre les bords supérieur 16 et inférieur 17 de la monture 2 en étant reliées à ces bords supérieur 16 et inférieur 17 par respectivement une première charnière supérieure 18 et une première charnière inférieure 19, ce dont il résulte que la pièce pivot 15 est montée pivotante relativement à la monture 2 autour d'un premier axe de pivotement A passant par ces première 18 et seconde 19 charnières supérieures.

L'axe de pivotement A est perpendiculaire à l'axe principal XX' du masque qui s'étend depuis l'arrière vers l'avant du masque 1.

La partie convexe avant de la pièce pivot 15 est reliée à une pièce de liaison en plastique 20 qui est solidaire de l'écran 5 au moyen d'une seconde charnière supérieure 21 et d'une seconde charnière inférieure 22, ce dont il résulte que la pièce pivot 15 est également montée pivotante relativement à l'écran 5 autour d'un second axe de pivotement B parallèle au première axe de pivotement A et donc également perpendiculaire à l'axe principal XX' du masque 1.

Il est à noter que la pièce de liaison 20 est solidarisée à l'écran 5 par tous moyens appropriés, par exemple par collage ou par encliquetage. Cette pièce de liaison 25 permet notamment de conférer l'épaisseur nécessaire pour l'aménagement des charnières de liaison 21,22 avec la pièce pivot 15.

Le masque de ski 1 comporte ainsi deux pièces pivot 15 situées respectivement sur les côtés gauche G et droit D du masque 1 en assurant un double pivotement, le premier entre la pièce pivot 15 et la monture 2, et le second entre la pièce pivot 15 et l'écran 5.

Le pivotement de la pièce pivot 15 relativement à la monture 2 et autour de l'axe de pivotement A permet à toute la surface de l'écran 5 de se translater selon la flèche F qui s'étend dans l'axe principal XX' du masque 1 pour être mis à distance de la monture 2. L'aération peut ainsi s'effectuer en partie supérieure de l'écran 5 selon la flèche F1 et en partie inférieure de l'écran 5 selon la flèche F2, ce qui génère un double flux d'air suffisant pour éviter à l'écran d'être couvert de buée lorsque l'utilisateur est à l'arrêt ou dans une phase de moindre effort et que le volume interne du masque est soumis aux effets de sa transpiration.

L'obtention de cette seconde position d'aération est ainsi obtenue par ce pivotement de la pièce pivot 15 relativement à la monture 2.

Pour ce qui est du second pivotement entre la pièce pivot 15 et l'écran 5, ce pivotement permet d'augmenter le débattement de l'écran 5 relativement à la monture 2 en créant un écartement E pouvant aller jusqu'à 15 millimètres, voire plus selon le dimensionnement des différentes pièces et ce, afin que l'écran 5 ne soit pas couvert de buée. En d'autres termes, l'accessoire peut prévoir un unique pivotement de la pièce pivot 15 relativement à la monture 2, le second pivotement apportant un débattement plus important pour un confort supplémentaire en terme d'efficacité d'aération.

Les moyens d'écartement 15 prévoient par ailleurs des moyens de maintien 23 de l'écran 5 en première position d'utilisation dans laquelle l'écran 5 est en appui de contact avec la monture 2.

Comme visible sur la figure 3, un bosselage 24 est ménagé sur le bord supérieur 16 de la monture 2, lequel bosselage 24 coopère avec une gorge non visible ménagée sur le bord latéral arrière 25 de la pièce de liaison 20 pour permettre l'encliquetage de cette pièce de liaison 20 sur la monture 2. Un même couple de bosselage 24 et gorge est également ménagé en partie inférieure des moyens d'écartement 15 ainsi que, par symétrie, en parties supérieure et inférieure des moyens d'écartement identiques situés sur le côté droit D du masque 1.

Il est en outre à noter que la configuration des différents pièces principales à savoir l'écran 5, les pièces de liaison gauche et droite 20, les pièces pivot gauche et droite 15, la monture 2 et la pièce d'appui 4 sont configurées de façon à permettre leur encastrement respectif lorsque l'écran 5 est dans la première position d'utilisation illustrée sur la figure 1, en assurant dans cette même position une continuité entre l'écran 5, la pièce de liaison 20 et la monture 2.

L'agencement tel que précédemment défini de la monture 2, de l'écran 5, des moyens d'écartement 15 et de la pièce de liaison 20 formant l'accessoire de l'invention, peuvent trouver application dans d'autres types de masques, notamment des masques professionnels de soudure ou de protection de façon générale.

## Revendications

1. Accessoire de protection visuelle comportant une monture sur laquelle est monté un écran, et qui comporte des moyens d'écartement (15) de l'écran (5) vis-à-vis de la monture (2) aptes à faire adopter à l'écran (5) une première position d'utilisation dans laquelle l'écran (5) est en appui de contact sur la monture (2), et une seconde position d'aération dans laquelle toute la surface de l'écran (5) est mise à distance de la monture (2) du côté opposé au visage de l'utilisateur, **caractérisé en ce que** lesdits moyens d'écartement comportent deux pièces pivot (15) situées respectivement au niveau du bord latéral gauche (G) et du bord latéral droit (D) du dit accessoire, chacune des pièces pivot (15) étant montée pivotante relativement à la monture (2) et pivotante relativement à l'écran (5).

2. Accessoire selon la revendication 1, **caractérisé en ce que** chacune des deux pièces pivot (15) comporte une première charnière supérieure (18) et une première charnière inférieure (18) de liaison pivotante à la monture (2).

3. Accessoire selon l'une quelconque des revendications 1 et 2, **caractérisé en ce que** chacune des deux pièces pivot (15) comporte une seconde charnière supérieure (21) et une seconde charnière Inférieure (22) de liaison pivotante à l'écran (5).

4. Accessoire selon l'une quelconque des revendications précédentes, **caractérisé en ce qu'**il comporte deux pièces de liaison (20) situées respectivement au niveau du bord latéral gauche (G) et du bord latéral droit (D) de l'écran (5) en étant d'une part solidarisé à l'écran (5) et d'autre part en liaison pivotante avec la pièce pivot associée (15).

5. Accessoire selon la revendication 4, **caractérisé en ce qu'**il comporte des moyens de maintien fixe (23) de l'écran (5) contre la monture (2) en position d'utilisation, les dits moyens de maintien fixe (23) comprenant au moins un bosselage (24) ménagé sur la monture (2) ou sur la pièce de liaison (20) qui coopère en encliquetage avec une gorge ménagée sur la monture (2) ou la pièce de liaison (20).

6. Masque de ski, **caractérisé en ce qu'**il comporte un accessoire selon l'une quelconque des revendications 1 à 5

## Patentansprüche

1. Visuelles Schutzzubehör, umfassend ein Brillengestell, auf dem ein Schirm montiert ist und der Beabstandungsmittel (15) des Schirms (5) gegenüber dem Brillengestell ((2) umfasst, die geeignet sind, den Schirm (5) eine erste Nutzungsposition, in der der Schirm (5) aufstützend mit dem Brillengestell (2) in Kontakt kommt, und eine zweite Belüftungsposition, in der die gesamte Oberfläche des Schirms (5) von dem Brillengestell (2) auf der entgegengesetzten Seite des Gesichts des Nutzers beabstandet ist, einnehmen zu lassen, **dadurch gekennzeichnet, dass** die genannten Beabstandungsmittel zwei Schwenkstücke (15) umfassen, die jeweils am linken seitlichen Rand (G) und am rechten seitlichen Rand (D) des genannten Zubehörs angeordnet sind, wobei jedes der Schwenkstücke (15) schwenkbar in Bezug auf das Brillengestell (2) und schwenkbar in Bezug auf den Schirm (5) montiert ist.

2. Zubehör gemäß Anspruch 1, **dadurch gekennzeichnet, dass** jedes der zwei Schwenkstücke (15) ein erstes oberes Scharnier (18) und ein erstes unteres Scharnier (19) zur Schwenkverbindung mit dem Brillengestell (2) umfasst.

3. Zubehör gemäß irgendeinem der Ansprüche 1 und 2, **dadurch gekennzeichnet, dass** jedes der zwei Schwenkstücke (15) ein zweites oberes Scharnier (21) und ein zweites unteres Scharnier (22) zur Verbindung mit dem Schirm (5) umfasst.

4. Zubehör gemäß irgendeinem der voranstehenden Ansprüche, **dadurch gekennzeichnet, dass** es zwei Verbindungsstücke (20) umfasst, die jeweils am linken seitlichen Rand (G) und am rechten seitlichen Rand (D) des Schirms (5) angeordnet und dabei einerseits fest mit dem Schirm (5) verbunden und andererseits schwenkbar mit dem zugeordneten Schwenkstück (15) verbunden sind.

5. Zubehör gemäß Anspruch 4, **dadurch gekennzeichnet, dass** es Haltemittel (23) des Schirms (5) gegen das Brillengestell (2) in der Nutzungsposition umfasst, wobei die genannten Haltemittel (23) wenigstens eine Erhebung (24) umfassen, die auf dem Brillengestell (2) oder auf dem Verbindungsstück (20) ausgespart ist, das per Einrasten mit einer Auskehlung auf dem Brillengestell (2) oder dem Verbindungsstück (20) zusammenwirkt.

6. Skimaske, **dadurch gekennzeichnet, dass** sie ein Zubehör gemäß irgendeinem der Ansprüche 1 bis 5 umfasst.

## Claims

1. A visual protection accessory including a frame on which a shield is mounted, and which includes means (15) for spacing the shield (5) from the frame (2) able to make the shield (5) assume a first use position in which the shield (5) leans in contact on the frame (2), and a second venting position in which the whole surface of the shield (5) is brought away from the frame (2) on the opposite side to the user's face, **characterised in that** said spacing means include two pivot parts (15) respectively located at the left lateral edge (G) and the right lateral edge (D) of said accessory, each of the pivot parts (15) being pivotally mounted with respect to the frame (2) and pivoting with respect to the shield (5).

2. The accessory according to claim 1, **characterised in that** each of both pivot parts (15) includes a first upper hinge (18) and a first lower hinge (19) for a pivoting connection to the frame (2).

3. The accessory according to any of claims 1 and 2, **characterised in that** each of both pivot parts (15) includes a second upper hinge (21) and a second lower hinge (22) for a pivoting connection with the shield (5).

4. The accessory according to any of the previous claims, **characterised in that** it includes two connecting parts (20) respectively located at the left lateral edge (G) and the right lateral edge (D) of the shield (5) by being on the one hand interlocked with the shield (5) and on the other hand in pivoting connection with the associated pivot part (15).

5. The accessory according to claim 4, **characterised in that** it includes means for fixedly holding (23) the shield (5) against the frame (2) in the use position, said fixed holding means (23) comprising at least one boss (24) provided on the frame (2) or on the connecting part (20) which cooperates as a snap-fit with a groove provided on the frame (2) or the connecting part (20).

6. Ski goggles, **characterised in that** they include an accessory according to any of claims 1 to 5.
